# EUROPÄISCHE PATENTSCHRIFT

(11) **EP 0 909 750 B1**
(45) Veröffentlichungstag und Bekanntmachung des Hinweises auf die Patenterteilung: **18.09.2002**
(21) Anmeldenummer: 98119215.6
(22) Anmeldetag: 12.10.1998
(51) Int. Cl.: C07C 45/67, C07C 49/203, B01J 31/22, C07F 5/06

(54) **Verfahren zur Herstellung Gamma, Delta-ungesättigter Ketone durch Carroll-Reaktion, neue Katalysatoren für dieses Verfahren und deren Herstellung**
Process for the preparation of gamma, delta-unsaturated ketones ba Carroll-reaciton, catalysts for this process and their preparation
Procédé pour la préparation de cétones gamma, delta-insaturées par la réaction Caroll, catalyseurs pour ce procédé et leur préparation

(30) Priorität: 17.10.1997 DE 19745672; 08.04.1998 DE 19815810
(43) Veröffentlichungstag der Anmeldung: 21.04.1999
(73) Patentinhaber: BASF AKTIENGESELLSCHAFT, 67056 Ludwigshafen (DE)
(72) Erfinder: Oost, Carsten Dr., 67098 Bad Dürkheim (DE); Stroezel, Manfred, 68549 Ilvesheim (DE); Etzrodt, Heinz Dr., 67434 Neustadt (DE); Bockstiegel, Bernhard Dr., 67354 Römerberg (DE); Jaedicke, Hagen Dr., 67069 Ludwigshafen (DE); Weller, Dietmar Dr., 67067 Ludwigshafen (DE); Laupichler, Lothar Dr., 67227 Frankenthal (DE); Reimer, Klaus, 67112 Mutterstadt (DE)

(56) Entgegenhaltungen:
- FR-A- 1 247 003
- FR-A- 1 265 113
- FR-A- 2 371 411
- GB-A- 822 572
- MEHROTRA R K ET AL: "Reactions of aluminum alkoxides with acetylacetone, benzoylacetone, and ethyl acetoacetate" CANADIAN JOURNAL OF CHEMISTRY, Bd. 39, 1961, Seiten 795-798, XP002091652 Ottawa
- Chem. Abstr., AN 88:75419 & JP-A-52112698 (21.09.1977)

## Beschreibung

Die Erfindung betrifft ein verbessertes Verfahren zur Herstellung γ,δ-ungesättigter Ketone, insbesondere von Methylheptenon, Geranylaceton und Farnesylaceton oder deren Dihydroderivate durch eine Carroll-Reaktion in Gegenwart von neuen Aluminiumkatalysatoren sowie neue geeignete Aluminiumkatalysatoren und deren Herstellung.

Unter einer Carroll-Reaktion versteht man die Kettenverlängerung eines Allyl- oder Propargylalkohols mit Acetessigestern oder Diketen unter Bildung von γ,δ-ungesättigten Ketonen. Sie kann beispielsweise nach folgendem Reaktionsschema verlaufen:

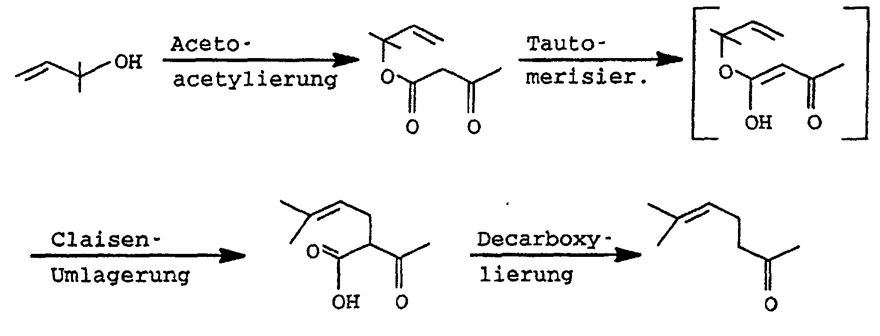

Im Schlüsselschritt lagert also der aus dem Allyl- oder Propargylalkohol und dem Acetoessigsäureester oder Diketen gebildete neue Acetoessigester in einer Claisenumlagerung zur β-Ketosäure um, die dann spontan decarboxyliert. Erste Untersuchungen über Carroll-Reaktionen sind im J. Am. Chem. Soc. 65 (1943) 1992 - 1998 beschrieben.

Seit dem Beginn der 50er Jahre wird diese Reaktion vielfältig bei der Herstellung von Terpenen angewendet. Beispielsweise werden die als essentielle Vorprodukte für Vitamin A und Vitamin E benötigten Terpene 2-Methyl-2-hepten-6-on, Geranylaceton und Farnesylaceton, durch Carroll-Reaktion in industriellem Maßstab hergestellt.

Als Katalysatoren für Carroll-Reaktionen wurden gemäß dem Verfahren von US 2 795 617 Aluminiumalkoholate, insbesondere das Aluminiumisopropylat der Formel Al(O-CH(CH₃)₂)₃ in Mengen von 0,8 bis 2,5 Mol-% bezogen auf den als Ausgangsmaterial eingesetzten Alkohol, verwendet.

Gemäß dem Verfahren der GB 886 353 wurden für Carroll-Reaktionen Aluminiumkomplexe mit Acetylaceton oder Acetessigestern, wie das Aluminium-tri-(acetylacetonat), Aluminium-tri-(methylacetoacetat) oder Aluminium-tri-(ethylacetoacetat) als Katalysatoren eingesetzt, die durch Umsetzen eines Aluminiumsalzes mit einer wässrigen Lösung von Acetylaceton in Ammoniak bzw. durch Umsetzen von Aluminiumtrialkoxiden mit Acetoessigsäuremethyl- oder -ethylester unter Abdestillieren des freigesetzten Alkohols und Isolieren des ausgefallenen festen Aluminiumkatalysators erhalten wurden. Für eine technische Anwendung sind sowohl die Verwendung von Ammoniak als auch der Festoffcharakter der Aluminiumverbindung von Nachteil.

In einer mehr theoretischen Abhandlung von Mehrotra et al im Can. J. of Chem. 39 (1961), Seiten 795 - 798, über Aluminiumkomplexe wird neben der Herstellung von dreifach mit Acetylaceton, Benzoylessigsäureestern oder Acetessigsäureestern komplexierten Aluminiumverbindungen auch die Herstellung von Aluminiumkomplexen beschrieben, in denen nur einer oder zwei der Alkoxyreste der entsprechenden Aluminiumalkoholate durch Acetylaceton-, Benzoylessigsäureester- oder Acetoessigsäureestergruppen ersetzt sind. Hierbei wird der einzuführende Ligand (z.B. Acetessigsäureethylester) mit einer Lösung von Aluminiumtriisopropylat in Benzol vermischt und diese Mischung 3 Stunden unter Rückfluß gekocht. Nach Beendigung der Reaktion wird der entstandene Alkohol als Azeotrop mit Benzol abdestilliert. Das von Mehrotra beschriebene Verfahren ist für eine technische Herstellung von Aluminiumverbindungen nicht geeignet. Einerseits ist die Verwendung von Benzol prohibitiv für den Einsatz der Aluminiumverbindung in weiteren Verfahren, wie z.B. für den Einsatz als Katalysator bei der Herstellung von Riechstoffen oder Vorprodukten von Vitamin E. Andererseits erfordert das zweistufige Verfahren (Reaktion unter Rückfluß + Destillation) hohe Investitionskosten und ist somit wirtschaftlich unattraktiv.
Die Verwendung dieser Aluminiumkomplexe als Katalysatoren für Carroll-Reaktionen wird hierin nicht erwähnt.

In der Patentschrift FR-A-1265113 wird ein Verfahren zur Herstellung ungesättigter Ketone durch Caroll Reaktion in Gegenwart von Al-Verbindungen beansprucht.
Die beanspruchten Al-Verbindung enthalten mindestens eine Alkoxygruppe.

Bei Verfahren im großtechnischen Maßstab wird bislang im wesentlichen Aluminium-tri-isopropylat verwendet.

Nachteilig an den bisher verwendeten Aluminiumkatalysatoren ist, daß sie kristalline Verbindungen sind, wodurch ein - bei Verfahren in technischem Maßstab - aufwendiges Arbeiten mit Feststoffen notwendig ist. Außerdem ist nachteilig, daß sie in den Reaktanten nur schlecht löslich sind. So löst sich beispielsweise das Aluminium-tri-(methyl-acetoacetat) bei Raumtemperatur nur zu etwa 4 % in Acetessigsäureethylester und so gut wie gar nicht in den für die Terpenchemie besonders interessanten Einsatzstoffen, wie 2-Methyl-3-buten-2-ol, Linalool oder Nerolidol.

Das unter Normalbedingungen flüssige und daher besser zu handhabende Aluminium-tri-sek.-butylat wird technisch nicht als Katalysator für Carroll-Reaktionen eingesetzt, weil bei seiner Verwendung größere Anteile an Nebenprodukten und daher weniger gute Ausbeuten an den gewünschten γ,δ-ungesättigten Ketonen erzielt werden.

Es war daher die Aufgabe der Erfindung, Katalysatoren für die Herstellung γ,δ-ungesättigter Ketone zu entwickeln, die leicht herstellbar sind, mindestens ebenso wirksam sind wie die für diese Reaktion bekannten Katalysatoren, in deren Gegenwart nur wenig Nebenprodukte gebildet werden, und die darüberhinaus auch in industriellem Maßstab technisch gut handhabbar sind. Als technisch gut handhabbar verstehen wir dabei, daß sie bei der Herstellung als Flüssigkeiten anfallen, als solche lagerstabil sind und als viskose Flüssigkeiten oder im Gemisch mit geringen Mengen eines niederen Alkohols oder eines anderen inerten Lösungsmittels pump- und fließfähig sind, so daß das in industriellem Maßstab aufwendige Arbeiten mit Feststoffen vermieden wird.

Es wurde nun überraschenderweise gefunden, daß alle obengenannten Anforderungen an Katalysatoren für die Carroll-Reaktion erfüllt sind, wenn man Aluminiumkatalysatoren verwendet, die mindestens einen Acetessigsäurealkyl- ester-Rest enthalten und 1 oder 2 Alkoxyreste oder aber ausschließlich Acetessigsäurealkylester-Reste enthalten, die mit sek.-Butanol oder Isobutanol oder aber mit mindestens zwei verschiedenen Alkanolen mit 1 bis 10 C-Atomen verestert sind.

Gegenstand der Erfindung ist daher ein Verfahren zur Herstellung von γ,δ-ungesättigten Ketonen der allgemeinen Formel I 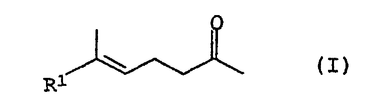 durch Umsetzen eines Allylalkohols der allgemeinen Formel II 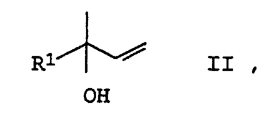 in der R¹ für H oder einen gesättigten oder ungesättigten verzweigten Kohlenwasserstoffrest mit 1 bis 20 C-Atomen steht, mit Diketen oder einem Acetessigsäurealkylester der allgemeinen Formel III 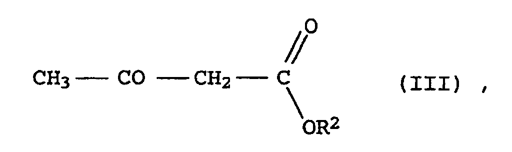 in der R² für Alkyl mit 1 bis 5 C-Atomen steht, in einer gegebenenfalls modifizierten Carroll-Reaktion in Gegenwart von einem Aluminiumkatalysator.

Unter bei Raumtemperatur stabilen, flüssigen Aluminiumverbindungen, die ausschließlich aus Acetessigsäureestern gebildeten Reste enthalten, verstehen wir allgemein Aluminiumverbindungen oder Gemische aus Aluminiumverbindung der allgemeinen Formel V 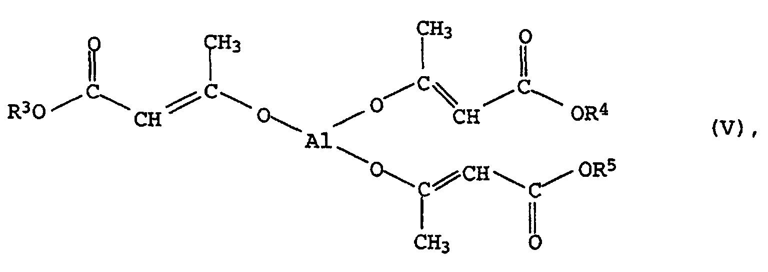 in der
- R³: für eine Alkylgruppe mit 1-5 C-Atomen vorzugsweise -CH₃, -C₂H₅ oder -CH(CH₃)-C₂H₅ steht,
- R⁴: für eine Alkylgruppe mit 3 bis 10 C-Atomen vorzugsweise -CH(CH₃)₂, -CH(CH₃)-C₂H₅ oder -CH(CH₃)-C₃H₇ steht und
- R⁵: für eine Alkylgruppe mit 1-10 C-Atomen vorzugsweise -CH₃, -C₂H₅, -CH(CH₃)₂, -CH(CH₃)-C₂H₅ oder -CH(CH₃)-C₃H₇ steht,
wobei die Reste R³, R⁴ und R⁵ nicht die gleiche Bedeutung haben dürfen.

Mit besonderem Vorteil gestaltet sich das erfindungsgemäße Verfahren, wenn man als Aluminiumkatalysatoren die neuen Gemische aus Aluminiumverbindungen der allgemeinen Formel V verwendet, 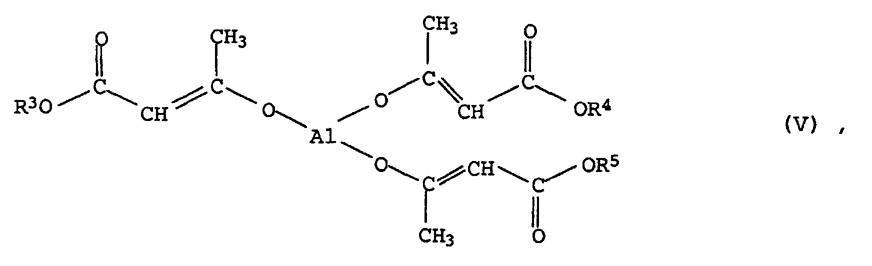 in der
- R³: für eine Alkylgruppe mit 1-5 C-Atomen, vorzugsweise -CH₃, -C₂H₅ oder -CH(CH₃)-C₂H₅ steht,
- R⁴: für eine Alkylgruppe mit 3 bis 10 C-Atomen, vorzugsweise
-CH(CH₃)₂, -CH(CH₃)-C₂H₅, -C(CH₃)₃ oder -CH(CH₃)-C₃H₇ steht und
- R⁵: für eine Alkylgruppe mit 1 bis 10 C-Atomen, vorzugsweise -CH₃, -C₂H₅, -CH(CH₃)₂, -CH(CH₃)-C₂H₅ oder -CH(CH₃) -C₃H₇ steht,
wobei die Reste R³, R⁴ und R⁵ nicht alle 3 die gleiche Bedeutung haben dürfen.

Gegenstand der Erfindung sind daher auch die neuen Gemische aus Aluminiumverbindungen der allgemeinen Formel V 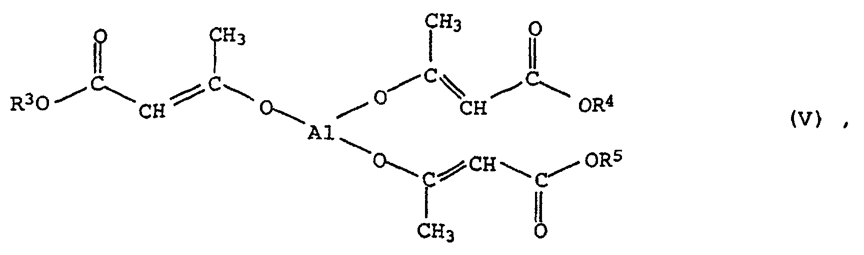 in der R³, R⁴ und R⁵ die oben angegebene Bedeutung haben, insbesondere Gemische aus Aluminiumverbindungen der allgemeinen Formel V, in der
- R³: für -CH₃, -C₂H₅ oder -CH(CH₃)-C₂H₅ steht,
- R⁴: für eine Alkylgruppe mit 3 bis 10 C-Atomen, vorzugsweise -CH(CH₃)₂, -CH(CH₃)-C₂H₅, -C(CH₃)₃ oder -CH(CH₃)-C₃H₇ steht und
- R⁵: für eine Alkylgruppe mit 1 bis 10 C-Atomen, vorzugsweise -CH₃, -C₂H₅, -CH(CH₃)₂, -CH(CH₃)-C₂H₅ oder -CH(CH₃)-C₃H₇ steht,
wobei die Reste R³, R⁴ und R⁵ nicht alle 3 die gleiche Bedeutung haben dürfen, sowie ein Verfahren zur Herstellung dieser Aluminiumverbindungen.

Als Allylalkohole der allgemeinen Formel II kommen Alkohole wie 2-Methyl-but-3-en-2-ol, 3,7-Dimethyl-oct-1,6-dien-3-ol (Linalool), 3,7-Dimethyl-oct-1-en-3-ol (6,7-Dihydrolinalool), 3,7,11-Trimethyl-dodeca-1,6,10-trien-3-ol (Nerolidol), 3,7,11,15-Tetramethyl-hexadeca-1,6,10,14-tetraen-3-ol (Geranyllinalool), 3,7,11,15-Tetramethyl-1-hexadecaen-3-ol (Isophytol), 3,7,11-Trimethyl-dodeca-1-en-3-ol und 3,7,11-Trimethyl-dodeca-1,6-dien-3-ol (10,11-Dihydro-nerolidol), insbesondere 2-Methyl-3-buten-2-ol, 6,7-Dihydro-linalool, Linalool, Nerolidol und 10,11-Dihydro-nerolidol in Betracht.

Als Acetessigsäurealkylester der allgemeinen Formel III für die Carroll-Reaktion kommen vor allem der Methylester, der Ethylester, der Isopropylester, der tert.-Butylester und der sek.-Butylester, insbesondere die kommerziell erhältlichen Acetessigsäuremethylester und Acetessigsäureethylester in Betracht.

Zur Durchführung der Carroll-Reaktion geht man im allgemeinen so vor, daß man den Allylalkohol der Formel II zunächst mit dem Aluminiumkatalysator und dann bei Temperaturen von 150 bis 220°C langsam mit dem Acetessigester der Formel III versetzt.

Bezüglich näherer Einzelheiten über die Durchführung von Carroll-Reaktionen verweisen wir auf W. Kimel et al. in J. Org. Chem. 23 (1958), Seiten 153 - 157 und J. Weichert et al. in Collect. Czech. Chem. Comm 25 (1960), Seiten 1914 bis 1921.

Als modifizierte Carroll-Reaktion bezeichnen wir in diesem Zusammenhang eine von Kimel und Sax in J. Org. Chem. 23, 2 (1958), Seiten 153 - 157 beschriebene Variante der Carroll-Reaktion, bei der die Allylalkohole der Formel II bei tiefen Temperaturen mit Diketen umgesetzt werden und anschließend die dabei erhaltenen, bis etwa 100°C stabilen Acetoacetate des Allylalkohols der Formel II bei Temperaturen von 150 bis 220°C zu den γ,δ-ungesättigten Ketonen umgelagert werden.

Zur Herstellung der erfindungsgemäß verwendeten Aluminiumkatalysatoren geht man mit Vorteil von Aluminium-tri-isopropylat, Aluminium-tri-sek.-butylat oder Aluminium-tri-sek.-pentylat aus, die ihrerseits in an sich bekannter Weise durch Umsetzen von metallischem Aluminium mit dem entsprechenden Alkohol in Gegenwart geeigneter Katalysatoren hergestellt werden. Man braucht die Aluminiumalkoholate nicht in isolierter und gereinigter Form einzusetzen. Nahezu gleich gute Erfolge erzielt man wenn man gleich das beim Umsetzen von Aluminium mit den entsprechenden Alkoholen erhaltene Reaktionsgemisch verwendet.

Für die Herstellung der neuen Gemische aus Aluminiumkatalysatoren der allgemeinen Formel V, in der R³ für eine Alkylgruppe mit 1 bis 5 C-Atomen, vorzugsweise für -CH₃ oder -C₂H₅ steht, R⁴ für eine Alkylgruppe mit 3 bis 10 C-Atomen, vorzugsweise -CH(CH₃)₂, -CH(CH₃)-C₂H₅, -C(CH₃)₃ oder -CH(CH₃)-C₃H₇ steht und R⁵ für eine Alkylgruppe mit 1 bis 10 C-Atomen, vorzugsweise für -CH₃, -C₂H₅, -CH(CH₃)-C₂H₅ oder -CH(CH₃)-C₃H₇ steht, wobei die Reste R³, R⁴ und R⁵ nicht alle 3 die gleiche Bedeutung haben dürfen,
geht man im allgemeinen so vor, daß man Aluminiumalkoholate der allgemeinen Formel VI

Al(O-R⁶)₃ (VI),

in der R⁶ für eine Alkylgruppe mit 2 bis 10 C-Atomen, vorzugsweise für -CH(CH₃)₂, -CH(CH₃)-C₂H₅, -C(CH₃) oder -CH(CH₃)-C₃H₇ steht, mit etwa 3 bis 10 Mol, vorzugsweise 3 bis 5 Mol, eines Acetessigsäurealkylesters eines von R⁶OH verschiedenen Alkohols mit 1 bis 5-C-Atomen, insbesondere 3 bis 5 Mol Acetessigsäuremethylester oder Acetessigsäureethylester pro Mol Aluminiumalkoholat erhitzt ohne während des Erhitzens den bei der Umsetzung freigesetzten höheren Alkohol aus dem Reaktionsgemisch zu entfernen. Im allgemeinen erhitzt man das Aluminiumalkoholat mit dem Acetessigsäurealkylester bei diskontinuierlicher Fahrweise unter dem sich allmählich einstellenden Rückfluß zum Sieden.

Um die Umsetzung zu beschleunigen, kann man das Erhitzen aber auch im geschlossenen System bei höheren Temperaturen vornehmen, was besonders für eine kontinuierliche Arbeitsweise wichtig ist. Für das Arbeiten unter Druck haben sich Temperaturen von 100 bis 220°C, vorzugsweise 150 bis 200°C, als vorteilhaft erwiesen.

Erhitzt man beispielsweise Aluminium-tri-isopropylat oder Aluminium-tri-sek.-butylat mit mindestens 3 Mol Acetessigsäuremethylester oder -ethylester pro Mol Aluminiumalkoholat auf Temperaturen von über 100°C, dann spaltet sich spontan der am Aluminium gebundene Alkohol ab und es bildet sich beim Abdestillieren des Alkohols das schwer lösliche Aluminium-tri-methylacetoacetat bzw. -triethylacetoacetat. Beläßt man dagegen den aus dem Aluminiumalkoholat freigesetzten Alkohol während des Erhitzens im Reaktionsgemisch und erhitzt weiter, dann verdrängt dieser Alkohol teilweise eine oder auch zwei der Alkoxygruppen des zunächst gebildeten Aluminium-tri-alkylacetoacetats. Es zeigte sich überraschenderweise, daß diese noch nicht beschriebenen Gemische aus Aluminiumacetoacetaten mit unterschiedlichen Estergruppen im Molekül nicht mehr kristallisieren, sondern als viskose Flüssigkeiten über Wochen stabil bleiben und so wesentlich besser technisch gehandhabt werden können als z.B. das kristalline und schwerlösliche Aluminium-tri-methylacetoacetat, und trotzdem eine gleich gute Wirksamkeit zeigen wie letzteres sowie eine bessere Wirksamkeit als Aluminium-tri-isopropylat (vgl. Beispiel 4a und Vergleichsbeispiel 4b). Die so hergestellten Triacetoacetate des Aluminiums stellen keine einheitlichen Verbindungen dar. Es handelt sich um Gemische aus beispielsweise Aluminium-di-methylacetoacetat-mono-sek.-butyl-acetoacetat und Aluminium-monomethylacetoacetat-di-sek.-butyl-acetoacetat. Als Voraussetzung für den flüssigen Aggregationszustand bei Raumtemperatur über mehrere Monate erwies sich der Grad der Umesterung, das heißt in diesem Fall das Verhältnis der Anzahl der 2-Butoxy-Gruppen zur Anzahl aller Alkoxygruppen, hier die Summe aus Methoxygruppen und 2-Butoxy-gruppen. Es müssen mehr als ca. jede dritte Methoxygruppe im Aluminium-tri-methylacetoacetat gegen die sek. -Amyloxygruppe, sek.-Butoxygruppe, tert.-Butoxygruppe oder iso-Propoxygruppe ausgetauscht sein um die erwünschten flüssig-viskosen Katalysatoren zu bilden.

Die neuen flüssig-viskosen Gemische aus Aluminiumkatalysatoren der allgemeinen Formel V, in der alle Alkoxyreste der Aluminium-tri-alkoxide durch zumindest teilweise unterschiedliche Acetessigsäurealkylesterreste ersetzt wurden, kann man auch dadurch erhalten, daß man die Aluminium-tri-alkoxide der allgemeinen Formel VI mit mindestens 3 Mol eines Gemisches aus mindestens 2 verschiedenen Acetessigsäurealkylestern, beispielsweise aus 2 Mol Acetessigsäuremethylester und 1 Mol Acetessigsäure-sek.-butylester, erhitzt.

Weiterhin wurde überraschenderweise gefunden, daß sich die flüssigen Aluminiumverbindungen der allgemeinen Formel V in denen ausschließlich aus Acetessigestern gebildete Reste enthalten sind, ganz besonders vorteilhaft kontinuierlich hergestellt werden können, wenn man Druck und Temperatur so wählt, daß ein Verdampfen des freigesetzten Alkohols aus dem Reaktionsgemisch nicht möglich ist.

Gegenstand der Erfindung ist daher auch ein Verfahren zur kontinuierlichen Herstellung der bei Raumtemperatur flüssigen Aluminiumverbindungen oder Gemischen aus Aluminiumverbindungen der allgemeinen Formel V, das dadurch gekennzeichnet ist, daß man je ein Mol eines Aluminiumalkoholats der allgemeinen Formel VI mit mindestens 3, vorzugsweise 3 bis 10 Molen eines Acetessigsäurealkylesters der allgemeinen Formel III oder aber mit mindestens 3 Mol eines Gemisches aus 2 oder 3 verschiedenen Acetessigsäurealkylestern der allgemeinen Formel III in reiner Form oder gelöst in einem geeigneten Lösungsmittel kontinuierlich
bei Temperaturen von 100 bis 250°C, vorzugsweise 150 bis 200°C,
einem Druck von 1 bis 100 bar, vorzugsweise 1 bis 10 bar, und
Verweilzeiten von 5 bis 120 Minuten, vorzugsweise 15 bis 45 Minuten umsetzt,
wobei Druck und Temperatur so gewählt werden müssen, daß sich im Reaktionsgefäß keine Gasphase ausbilden kann.

Die kontinuierliche Umsetzung muß überraschenderweise als Flüssigphasereaktion durchgeführt werden, d.h. eine Gasphase ist im Gegensatz zum diskontinuierlichen Verfahren nicht erlaubt. Das Reaktionsgemisch darf also nicht sieden. Führt man die Reaktion bei einem zu niedrigen Druck durch, so bildet sich eine Gasphase, in welcher sich der Alkohol mit dem niedrigeren Siedepunkt anreichert. Dieser wird hierdurch dem Gleichgewicht entzogen, so daß die Umesterungsreaktion nur unvollständig abläuft. Dies wiederum kann dazu führen, daß der Aluminiumkatalysator nicht flüssig ist, sondern ausfällt. Folglich muß der Druck so eingestellt werden, daß die vom Druck abhängige Siedetemperatur des Reaktionsgemisches über der frei wählbaren Reaktionstemperatur liegt.

Bei der Umsetzung handelt es sich um eine reversible Gleichgewichtsreaktion, wobei die Gleichgewichtslage und die Geschwindigkeit der Gleichgewichtseinstellung von der Temperatur abhängig sind. Temperatur und Verweilzeit können deshalb nicht unabhängig voneinander eingestellt werden. Bei einer gegebenen Temperatur muß die Verweilzeit so gewählt werden, daß ein ausreichender Umesterungsgrad erreicht wird. Ansonsten besteht die Gefahr, daß der Katalysator ausfällt.

Der Umesterungsgrad steigt überraschenderweise quadratisch mit der Temperatur aber nur linear mit der Verweilzeit an. Eine Änderung der Temperatur hat daher einen wesentlich stärkeren Einfluß auf den Umesterungsgrad als die Verweilzeit.

Bei hoher Temperatur und großer Verweilzeit wird ein Gleichgewichtszustand erreicht. Bei gegebener Temperatur ist daher nur sinnvoll, diejenige Verweilzeit zu wählen, in welcher der Gleichgewichtszustand bzw. der gewünschte Umesterungsgrad gerade erreicht wird. Eine weitere Erhöhung der Verweilzeit hat keinen Einfluß auf die Reaktion und führt somit nur zu einer Reduzierung der Raumzeitausbeute.

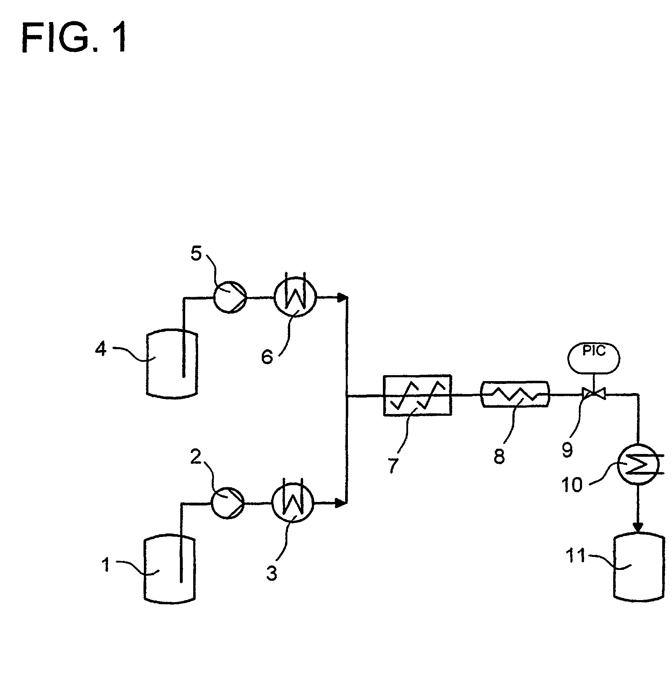

Bei der kontinuierlichen Herstellung der flüssigen Aluminiumverbindungen der allgemeinen Formel V geht man mit Vorteil so vor, wie es in Figur 1 (Fig. 1) schematisch dargestellt ist. Hierbei wird der Acetessigsäurealkylester der allgemeinen Formel III mit Hilfe einer Pumpe 2 aus einem Vorlagebehälter 1 und das Aluminiumalkoholat der allgemeinen Formel VI oder eine Lösung dieses Alkoholats mit Hilfe einer Pumpe 5 aus einem Vorlagebehälter 4 in den Mischer 7 gepumpt. Es ist von Vorteil, die beiden Reaktanten mit Hilfe der Wärmeaustauscher 3 bzw. 6 vorzuwärmen oder sogar auf die Reaktionstemperatur zu bringen. Der Mischer 7 muß bei erhöhter Temperatur und erhöhtem Druck betrieben werden. Nach der Vermischung der Reaktanten im Mischer 7 erfolgt vorteilhaft die kontinuierliche Umsetzung in einem Reaktor 8 mit Rohrcharakteristik. Das Reaktionssystem wird mit Hilfe einer Druckregelung 9 bei dem gewünschten Druck gehalten, wobei der Druck und die Temperatur so gewählt werden müssen, daß ein Verdampfen des aus dem Aluminiumalkohol freiwerdenden Alkohols nicht möglich ist. Das den Reaktor verlassende Reaktionsgemisch wird auf Normaldruck entspannt, mit Hilfe des Wärmetauschers 10 abgekühlt und in einem Vorlagbehälter 11 gesammelt.

Gegenstand der Erfindung ist dementsprechend auch ein Verfahren zur kontinuierlichen Herstellung der Aluminiumverbindungen der allgemeinen Formel V, wie es oben beschrieben ist, das dadurch gekennzeichnet ist, daß man das Aluminiumalkoholat der allgemeinen Formel VI mit einem Acetessigsäurealkylester bzw. den Acetessigsäurealkylestern der allgemein Formel III kontinuierlich unter erhöhtem Druck und erhöhter Temperatur in einem Mischer zusammenführt und anschließend in einem Reaktor mit Rohrcharakteristik umsetzt.

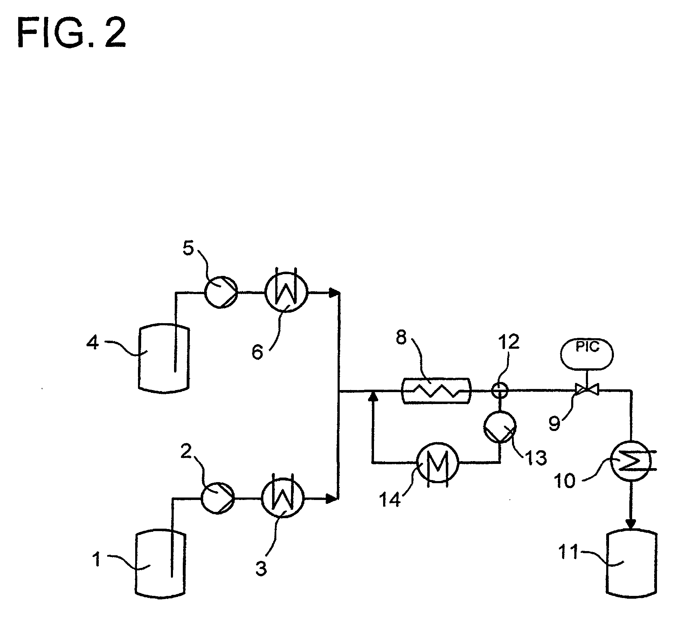

Alternativ zu dieser bevorzugten Ausführungsform ist es auch möglich die kontinuierliche Umsetzung in einem sogenannten Schlaufenreaktor oder Kreislaufreaktor durchzuführen, was praktisch bedeuten kann, daß man die Umsetzung in einem Strömungsrohrreaktor mit externem Kreislauf durchführen kann, wie es schematisch in Figur 2 (Fig. 2) dargestellt ist. Hierbei wird der den Reaktor 8 verlassende Reaktionsaustrag über einen Dreiwegehahn 12 mit einer Pumpe 13, gegebenenfalls über einen Wärmeaustauscher 14, zum Reaktoreingang zurückgeführt. Auf einen vorgeschalteten Mischer 7 kann in diesem Fall verzichtet werden.

Anstelle des Rohrreaktors oder des Schlaufenreaktors ist es jedoch prinzipiell auch möglich, die kontinuierliche Umsetzung in einem Rührkessel oder einer Rührkesselkaskade als Reaktionsgefäß durchzuführen.

Den bei der Umsetzung freigesetzten Alkohol kann man nach Abschluß der Umsetzung durch einstufige Destillation, beispielsweise mit Hilfe eines Dünnschichtverdampfers, aus dem Reaktionsgemisch entfernen.

Läßt man nach Reaktionsende den freigesetzten Alkohol im Reaktionsgemisch, so weist die resultierende Lösung in der Regel eine gute Fließ- und Pumpfähigkeit auf.

Zur Erzielung einer besseren Fließ- oder Pumpbarkeit kann man den flüssig viskosen Katalysatoren aber auch geringe Mengen, d.h. Mengen von 20 bis 100 Gew.-%, eines niederen Alkohols oder eines anderen inerten Lösungsmittels zusetzen.

In dem erfindungsgemäßen Verfahren verwendet man die Aluminiumkatalysatoren im allgemeinen in Mengen von etwa 0,5 bis 3 Mol-%, vorzugsweise etwa 1,0 bis 2,5 Mol-%, bezogen auf den eingesetzten Allylalkohol der Formel II.

Mit Hilfe des erfindungsgemäßen Verfahrens, insbesondere bei Verwendung der neuen gemischten Aluminium-trialkylacetoacetate der Formel V als Katalysatoren, können die als essentielle Vitaminvorprodukte benötigten Ketone der allgemeinen Formel I, wie 2-Methyl-2-hepten-6-on, Geranylaceton, (6,10-Dimethyl-5,9-undecadien-2-on), Dihydrogeranylaceton (6,10-Dimethyl-5-undecen-2-on), Farnesylaceton (6,10,14-Trimethyl-5,9,10-pentadecatrien-2-on), 6,10-Dimethyl-5-undecen-2-on und Dihydrofarnesylaceton (6,10,14-Trimethyl-5,10-pentadecadien-2-on), auf technisch einfache Weise in guten Ausbeuten und guten Qualitäten hergestellt werden.

### Beispiel 1

### Umsetzung von Al(O-CH(CH₃)-C₂H₅)₃ mit Acetessigsäuremethylester (AME)

a) In einem mit Rückflußkühler versehenen Reaktionsgefäß wurden 530 g reines Aluminium-tri-sek.-butylat auf 160°C erwärmt und hierzu bei dieser Temperatur innerhalb von zwei Stunden (h) unter Rühren gleichmäßig 670 g AME zugesetzt. In exothermer Reaktion wurden bei dieser Umsetzung sek.-Butanol und Methanol freigesetzt, aber diese nicht aus dem Reaktionsgemisch entfernt, sondern durch Rückflußkühlung im Reaktionsgemisch gehalten.
   Nach Zulaufende wurden über eine kurze Kolonne bei einem Rücklaufverhältnis von 10 : 1 Leichtsieder abdestilliert. Innerhalb von 12 h destillierten so 150,3 g Alkohol ab, der aus sek.-Butanol und Methanol bestand. Anschließend kühlte man das Reaktionsgemisch ab und destillierte unter vermindertem Druck (30 mbar) weiteren Alkohol ab. Es verblieben 860 g einer viskosen Flüssigkeit, aus der keine Kristalle ausfielen.
   Zur Herstellung einer gut pumpbaren, leicht beweglichen und stabilen Katalysatorlösung kann der Rückstand mit etwa 140 g Methanol versetzt werden.
b) Zum Vergleich
   530 g reines Aluminium-tri-sek.-butylat wurde auf 150°C erhitzt und danach wurden unter Rühren und gleichzeitigem Abdestillieren von Alkoholen innerhalb von 2 h 670 g AME zugepumpt. Nach Zulaufende destillierte man noch 0,5 h weiter und ließ dann auf 30°C abkühlen. Unter spontaner Kristallisation erstarrte der Rückstand zu einem Feststoffblock beste hend aus dem schwer löslichen Aluminium-tri-methylacetoacetat.

### Beispiel 2

### Umsetzung von Al(-O-CH(CH₃)₂)₃ mit AME

In einem mit Rückflußkühler versehenen Reaktionsgefäß wurden 106 g reines Al(-O-CH(CH₃)₂)₃ auf 180°C erhitzt und hierzu bei dieser Temperatur innerhalb von 3 h unter Rühren 180 g AME zugepumpt und anschließend noch 1 h unter Rückfluß zum Sieden erhitzt. Anschließend wurde analog Beispiel 1 der gebildete Alkohol abdestilliert. Man erhielt 199,5 g einer viskosen Flüssigkeit als Rückstand, bestehend aus einem Gemisch aus Aluminium-tri-(methyl-acetoacetat-iso-propylacetoacetat). Der so erhaltene Katalysator blieb 50 Tage und mehr flüssig und pumpbar.

### Beispiel 3

In einem mit Rückflußkühler versehenen Reaktionsgefäß wurden 50,3 g reines Al(-O-CH(CH₃)-C₂H₅)₃ auf 150°C erhitzt und hierzu unter Rühren und Erhitzen unter sich langsam einstellendem Rückfluß innerhalb von 1 h 72 g AME zugepumpt. Anschließend erhitzte man das Reaktionsgemisch noch 15 h unter Rückfluß und erhielt so 120 g einer dünnflüssigen haltbaren Katalysatorlösung enthaltend verschiedene Aluminium-tri-(methyl-acetoacetat-sek.-butyl-acetoacetate) neben sek.-Butanol und Methanol.

### Beispiel 4

a) 39 g Linalool (3,7-Dimethyl-oct-1,6-dien-3-ol) und 3,27 g der gemäß Beispiel 3 erhaltenen Katalysatorlösung mit einem Gehalt an 0,149 g Al (4,54 % in dem flüssigen Katalysator) wurden zusammen auf 180°C erhitzt. Sofort nach dem Erreichen dieser Temperatur wurden innerhalb von 2 h bei einer Innentemperatur von 180 ± 4°C gleichmäßig 30,45 g AME zugepumpt. Der Abgasstrom wurde gekühlt. Man isolierte 6,9 g Leichtsieder, man rührte noch 30 min bei 180°C, kühlte und destillierte unter vermindertem Druck. Man erhielt 46,55 g reines E,Z-6,10-Dimethyl-undeca-5,9-dien-2-on (Geranylaceton). Dies entspricht einer Ausbeute von 95 % der Theorie.
b) Zum Vergleich
   39 g Linalool und 1,122 g Aluminium-tri-isopropylat (mit einem Gehalt von 0,148 g A1) wurden zusammen auf 180°C erhitzt und analog Beispiel 4a) innerhalb von 2 h mit 30,45 g AME versetzt. Der Abgasstrom wurde gekühlt und dabei 5,6 g CH₃OH aufgefangen. Man rührte noch 30 min bei 180°C, kühlte und destillierte unter vermindertem Druck. Nach einem geringen Vorlauf isolierte man 43,23 g Geranylaceton. Dies entspricht einer Ausbeute von 88 % der Theorie.

### Beispiele 5 bis 12

### Kontinuierliche Herstellung im Rohrreaktor

### a) Beschreibung der Reaktionsapparatur (Schema siehe Figur 1)

Die in Tabelle 1 näher erläuterten Umsetzungen wurden in einem beheizbaren Rohrreaktor 8 , der aus zwei 6 Meter (m) langen Rohrschlangen mit einem Innendurchmesser von 6 mm bestand (Reaktorvolumen dementsprechend 340 ml), durchgeführt. Aluminium-trisek.-butylat (100 %ig, d.h. ohne Lösungsmittel; Dichte 0,97 g/cm³) und Acetessigsäuremethylester (Dichte 1,077 g/cm³) wurden mit Hilfe der Zahnradpumpen 2, bzw. 5, aus den Vorratsbehältern 1, bzw.4, über die durch Wärmeaustauscher 3, bzw. 6 beheizten Leitungen in den beheizbaren Mischer 7 gefördert. Die beiden Reaktanten wurden in dem Mischer 7 bei Reaktionstemperatur intensiv vermischt und anschließend in den oben beschriebenen Rohrreaktor überführt. Hinter dem Reaktorausgang befand sich die Druckregelung 9, über die der gewünschte Systemdruck eingestellt wurde, sowie eine Kühlstrecke 10 , in welcher das Produkt auf Raumtemperatur (RT) abgekühlt wurde. Anschließend wurde das Produkt in dem Vorlagebehälter 11 gesammelt und nach destillativer Abtrennung der Leichtsieder mittels ¹H-NMR-Spektroskopie der Umesterungsgrad bestimmt.

Als Umesterungsgrad wurde dabei das Verhältnis der Anzahl der 2-Butoxy-gruppen zur Anzahl aller Alkoxygruppen, d.h. der Summe von Methoxygruppen und 2-Butoxy-gruppen, definiert.

### b) allgemeine Beschreibung der Umsetzungen

In der oben beschriebenen Apparatur wurden die aus der folgenden Tabelle 1 ersichtlichen Mengen an Acetessigsäuremethylester (AME) und Aluminium-tri-sek.-butylat (Al(OsekBu)₃) bei der aus der Tabelle 1 ersichtlichen Temperatur und dem aus der Tabelle 1 ersichtlichen Druck kontinuierlich umgesetzt, wobei die aus der Tabelle 1 ersichtliche Verweilzeit eingestellt wurde. Das erhaltene Produkt wurde nach Abtrennung der Leichtsieder (Methanol, 2-Butanol, Acetessigsäuremethylester) mittels ¹H-NMR-Spektroskopie analysiert.

Der jeweils erzielte Umesterungsgrad sowie der Aluminiumgehalt sind in der Tabelle 1 angegeben.

Das Produkt blieb auch nach einer Lagerzeit von mehreren Monaten flüssig und ist somit vorteilhaft als Katalysator für Carroll-Reaktionen einsetzbar.

Lediglich in Beispiel 10 reichten der Reaktionsdruck in Verbindung mit der Verweilzeit nicht aus, um einen ausreichenden Umesterungsgrad zu erzielen.

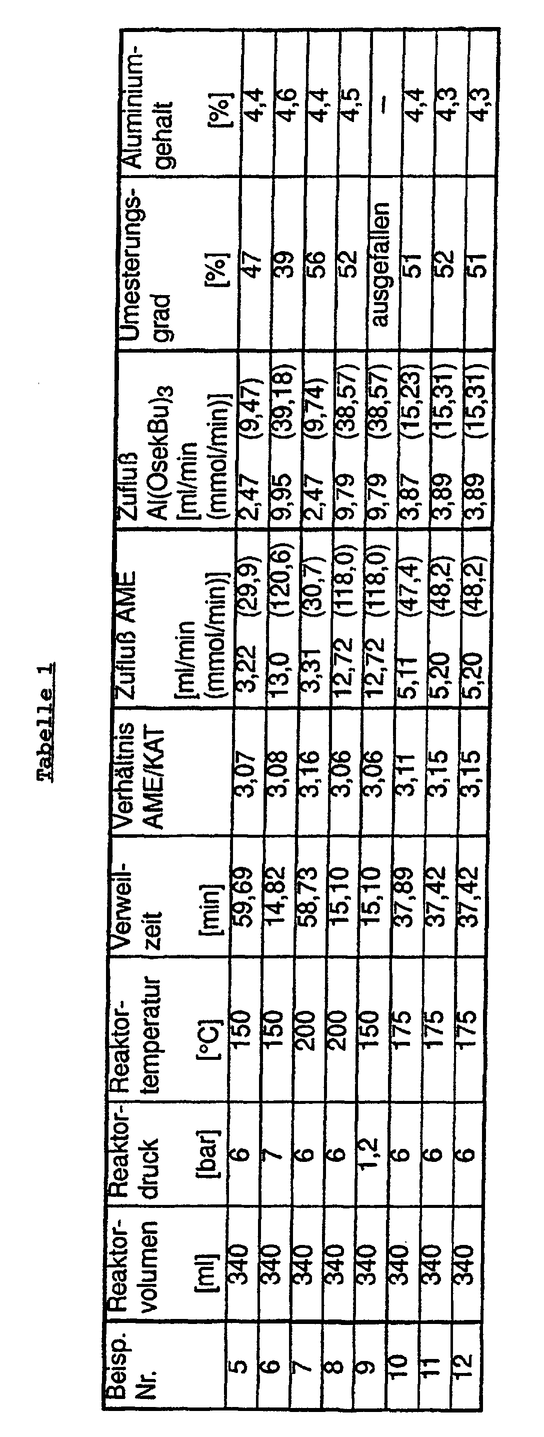

### Beispiele 13 und 14

### Vergleich der kontinuierlichen Herstellung im Rohrreaktor und im Schlaufenreaktor

### a) Umsetzung im Rohrreaktor (Schema siehe Figur 1)

In der oben beschriebenen Apparatur wurden 5.3 g/min (45.7 mmol/min) Acetessigsäuremethylester mit 3.8 g/min (15.5 mmol/min) Aluminium-tri-sek.-butylat bei einer Temperatur von 175°C und einem Druck von 6 bar kontinuierlich umgesetzt. Als Reaktor wurde der oben beschriebene Rohrreaktor verwendet, wobei eine Verweilzeit von 38 Minuten eingestellt wurde. Nach Abtrennung der Leichtsieder (Methanol, 2-Butanol, Acetessigsäuremethylester) wurde das Produkt mittels ¹H-NMR-Spektroskopie analysiert.

Der Umesterungsgrad betrug 51% und der Aluminiumgehalt 4.4 %. Das Produkt blieb auch nach einer Lagerzeit von mehreren Monaten flüssig.

### b) Umsetzung im sogenannten Kreislauf- oder Schlaufenreaktor (Schema siehe Figur 2)

Die Umsetzung wurde unter denselben Reaktionsbedingungen (45.7 mmol/min Acetessigsäure-methylester, 15.5 mmol/min Aluminium-trisek.-butylat, Reaktionstemperatur = 175°C, Druck = bar, Verweilzeit = 38 min) durchgeführt, ,jedoch wurde der obenbeschriebene Reaktor durch Rückführung des den Reaktor verlassenden Reaktionsgemisches über einen Zweiwegehahn 12, die Pumpe 13 und die durch den Wärmeaustauscher 14 beheizte Leitung in den Reaktoreingang in einen sogenannten Schlaufenreaktor umgewandelt. Es wurde ein Kreislaufverhältnis von ca. 20 eingestellt.

Der Umesterungsgrad betrug hierbei 48% und ist damit niedriger als der im Rohreaktor erzielte Wert, der Aluminiumgehalt betrug 4.5 %. Auch dieses mit dem Schlaufenreaktor hergestellte Produkt war nach einer Lagerzeit von mehreren Monaten noch flüssig und somit vorteilhaft als Katalysator für Carroll-Reaktionen einsetzbar.

### Beispiel 15

### Umsetzung in einem kontinuierlichen Rührkessel (CSTR)

In der oben beschriebenen Apparatur wurden der Mischer und der Rohrreaktor durch einen Rührkessel mit einem Reaktionsvolumen von 60 ml ersetzt. Anschließend wurden 0.74 g/min (6.4 mmol/min) Acetessigsäuremethylester (AME) mit 0.68 g/min (2.13 mmol/min ber. 100%) einer 77%igen Lösung von Aluminium-tri-sek.-butylat (Al(OsekBu)₃) in 2-Butanol bei einer Temperatur von 150°C und einem Druck von 65 bar kontinuierlich umgesetzt. Die Verweilzeit betrug 42 Minuten. Nach Abtrennung der Leichtsieder wurde das Produkt mittels ¹H-NMR-Spektroskopie analysiert. Es wurde ein Umesterungsgrad von 39 % erreicht. Dieser Wert liegt deutlich unter den im Rohrreaktor erzielten Werten. Dennoch wurde ein bei Raumtemperatur stabiler flüssiger Aluminiumkatalysator erhalten.

## Patentansprüche

1. Verfahren zur Herstellung von γ,δ-ungesättigten Ketonen der allgemeinen Formel I 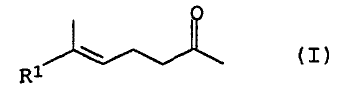 durch Umsetzen eines Allylalkohols der allgemeinen Formel II 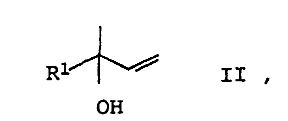 in der R¹ für H oder einen gesättigten oder ungesättigten verzweigten Kohlenwasserstoffrest mit 1 bis 20 C-Atomen steht, mit Diketen oder einem Acetessigsäurealkylester der allgemeinen Formel III 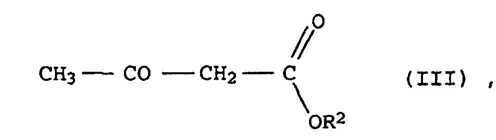 in der R² für Alkyl mit 1 bis 5 C-Atomen steht, in einer gegebenenfalls modifizierten Carroll-Reaktion in Gegenwart von einem Aluminiumkatalysator, **dadurch gekennzeichnet, daß** man als Aluminiumkatalysator eine Aluminiumverbindung der allgemeinen Formel V oder ein Gemisch aus Aluminiumverbindungen der allgemeinen Formel V verwendet, 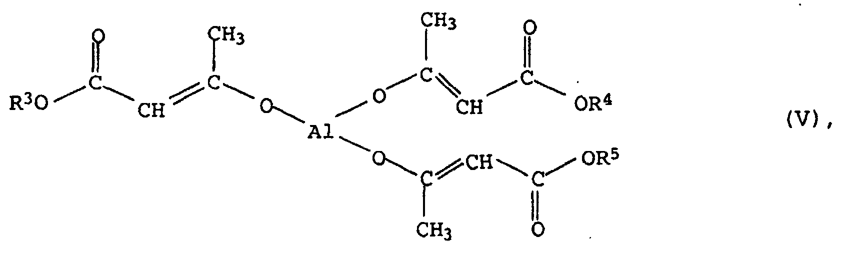 in der
R³ für eine Alkylgruppe mit 1-5 C-Atomen,
R⁴ für eine Alkylgruppe mit 3 bis 10 C-Atomen, und
R⁵ für eine Alkylgruppe mit 1-10 C-Atomen steht,
wobei die Reste R³, R⁴ und R⁵ nicht gleich sein dürfen.

2. Verfahren gemäß Anspruch 1, **dadurch gekennzeichnet, daß** man ein Gemisch aus Aluminiumverbindungen der allgemeinen Formel V 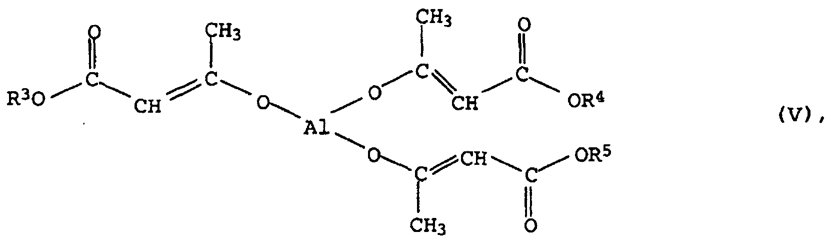 in der
R³ für eine Alkylgruppe mit 1-5 C-Atomen steht,
R⁴ für eine Alkylgruppe mit 3 bis 10 C-Atomen steht und
R⁵ für eine Alkylgruppe mit 1-10 C-Atomen steht,
wobei die Reste R³, R⁴ und R⁵ nicht alle 3 die gleiche Bedeutung haben dürfen, als Aluminiumkatalysator verwendet.

3. Verfahren gemäß Anspruch 1, **dadurch gekennzeichnet, daß** man als Allylalkohol der allgemeinen Formel II 2-Methyl-3-buten-2-ol, Linalool, 6,7-Dihydro-linalool, Nerolidol, 10,11-Dihydro-nerolidol oder Geranyllinalool mit dem Acetessigsäurealkylester der Formel III umsetzt.

4. Gemische aus bei Raumtemperatur flüssigen Aluminiumverbindungen der allgemeinen Formel V 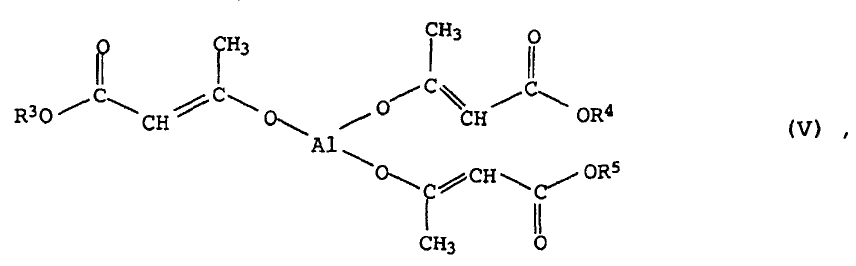 in der
R³ für eine Alkylgruppe mit 1-5 C-Atomen steht,
R⁴ für eine Alkylgruppe mit 3 bis 10 C-Atomen steht und
R⁵ für eine Alkylgruppe mit 1-10 C-Atomen steht,
wobei die Reste R³, R⁴ und R⁵ nicht alle 3 die gleiche Bedeutung haben dürfen.

5. Verfahren zur Herstellung der bei Raumtemperatur flüssigen Gemische aus Aluminiumverbindungen der allgemeinen Formel V gemäß Anspruch 4, **dadurch gekennzeichnet, daß** man Aluminiumalkoholate der allgemeinen Formel VI
Al (O-R⁶)₃ (VI),
in der R⁶ für eine Alkylgruppe mit 2 bis 10 C-Atomen steht,
mit mindestens 3 Mol eines Acetessigsäurealkylesters eines von R⁶OH verschiedenen Alkohols pro Mol Aluminiumalkoholat erhitzt ohne während des Erhitzens den bei der Umsetzung freigesetzten Alkohol aus dem Reaktionsgemisch zu entfernen.

6. Verfahren zur Herstellung der bei Raumtemperatur flüssigen Gemische aus Aluminiumverbindungen der allgemeinen Formel V gemäß Anspruch 4, **dadurch gekennzeichnet, daß** man Aluminiumalkoholate der allgemeinen Formel VI
Al (O-R⁶)₃ (VI),
in der R⁶ für eine Alkylgruppe mit 2 bis 10 C-Atomen steht, mit mindestens 3 Mol eines Gemisches aus mindestens 2 verschiedenen Acetessigsäurealkylestern erhitzt.

7. Kontinuierliches Verfahren zur Herstellung der bei Raumtemperatur flüssigen Gemische aus Aluminiumverbindungen der allgemeinen Formel V, gemäß Anspruch 4, **dadurch gekennzeichnet, daß** man je ein Mol eines Aluminiumalkoholats der allgemeinen Formel VI
Al (O-R⁶)₃ (VI),
in der R⁶ für eine Alkylgruppe mit 2 bis 10 C-Atomen und 3 bis 10 Mol eines Acetessigsäurealkylesters der allgemeinen Formel II
CH₃-CO-CH₂-COOR² (III),
in der R² für Alkyl mit 1 bis 5 C-Atomen steht,
wobei R² und R⁶ verschieden sind oder aber mit mindestens 3 Mol eines Gemisches aus 2 oder 3 verschiedenen Acetessigsäurealkylestern der allgemeinen Formel III in reiner Form oder gelöst in einem geeigneten inerten Lösungsmittel kontinuierlich
bei Temperaturen von 100 bis 250°C,
einem Druck von 1 bis 100 bar und
Verweilzeiten von 5 bis 120 Minuten umsetzt,
wobei der Druck und die Temperatur so gewählt werden müssen, daß sich im Reaktionsgefäß keine Gasphase ausbilden kann.

8. Verfahren gemäß Anspruch 7, **dadurch gekennzeichnet, daß** man das Aluminiumalkoholat der allgemeinen Formel VI mit dem Acetessigsäurealkylester bzw. den Acetessigsäurealkylestern der allgemeinen Formel III kontinuierlich unter erhöhtem Druck und erhöhter Temperatur in einem Mischer zusammengeführt und anschließend in einem Reaktor mit Rohrcharakteristik umsetzt.

9. Verfahren gemäß einem der Ansprüche 1 bis 3, **dadurch gekennzeichnet, daß** R³ die Gruppe -CH₃, -C₂H₅ oder -CH(CH₃)-C₂H₅ bedeutet, R⁴ -CH(CH₃)₂, -CH(CH₃)-C₂H₅ oder -CH-(CH₃)C₃H₇ bedeutet R⁵ CH₃, -C₂H₅, CH(CH₃)₂, CH(CH₃)-C₂H₅ oder -CH-(CH₃)C₃H₇ bedeutet.

10. Verfahren gemäß einem der Ansprüche 5 bis 7, **dadurch gekennzeichnet, daß** R⁶ -CH₂-CH(CH₃)₂, -CH(CH₃)-C₂H₅ oder -CH(CH₃)C₃H₇ bedeutet.

11. Verfahren gemäß Anspruch 7, **dadurch gekennzeichnet, daß** R² -CH₃, -C₂H₅, -CH(CH₃)-C₂H₅ oder -CH₂-CH(CH₃)₂ bedeutet.

## Claims

1. A process for preparing γ,δ-unsaturated ketones of the general formula I 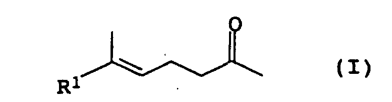 by reacting an allyl alcohol of the general formula II 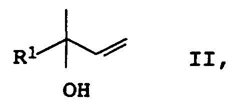 in which R¹ is H or a saturated or unsaturated branched hydrocarbon radical having 1 to 20 carbon atoms, with diketene or an alkyl acetoacetate of the general formula III 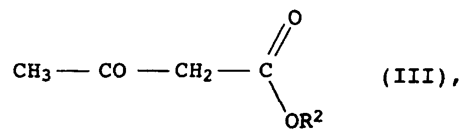 in which R² is alkyl having 1 to 5 carbon atoms, in an unmodified or modified Carroll reaction in the presence of an aluminum catalyst, wherein an aluminum compound of the general formula V or a mixture of aluminum compounds of the general formula V, 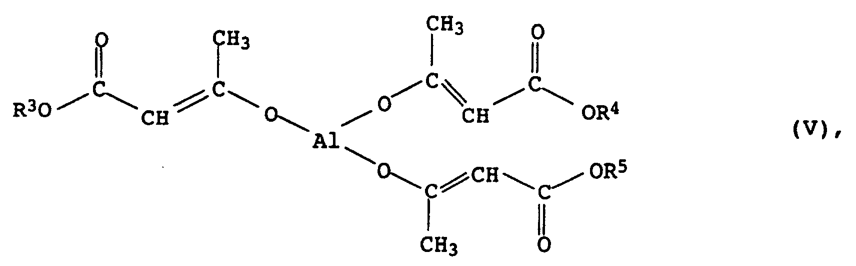 in which
R³ is an alkyl group having 1-5 carbon atoms,
R⁴ is an alkyl group having 3 to 10 carbon atoms, and
R⁵ is an alkyl group having 1-10 carbon atoms,
where the radicals R³, R⁴ and R⁵ must not be the same, is used as aluminum catalyst.

2. A process as claimed in claim 1, wherein a mixture of aluminum compounds of the general formula V 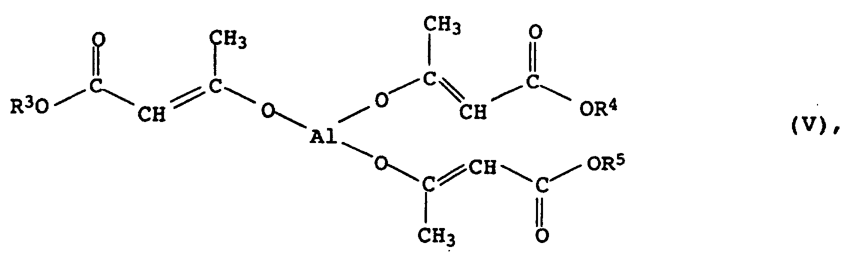 in which
R³ is an alkyl group having 1-5 carbon atoms,
R⁴ is an alkyl group having 3 to 10 carbon atoms, and
R⁵ is an alkyl group having 1-10 carbon atoms,
where all 3 of the radicals R³, R⁴ and R⁵ may not have the same meaning, is used as aluminum catalyst.

3. A process as claimed in claim 1, wherein 2-methyl-3-buten-2-ol, linalool, 6,7-dihydrolinalool, nerolidol, 10,11-dihydronerolidol or geranyllinalool is reacted as allyl alcohol of the general formula II with the alkyl acetoacetate of the formula III.

4. A mixture of aluminum compounds which are liquid at room temperature and have the general formula V 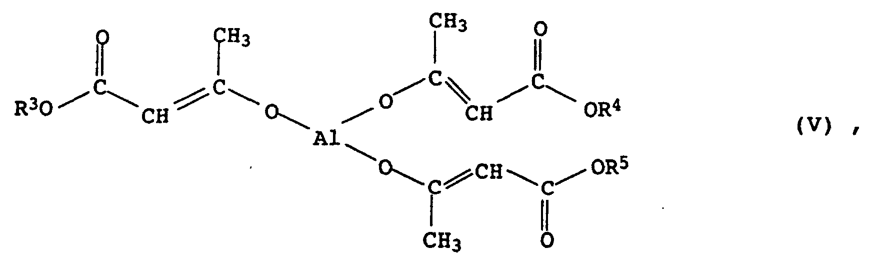 in which
R³ is an alkyl group having 1-5 carbon atoms,
R⁴ is an alkyl group having 3 to 10 carbon atoms, and
R⁵ is an alkyl group having 1-10 carbon atoms,
where all 3 of the radicals R³, R⁴ and R⁵ may not have the same meaning.

5. A process for preparing the mixtures, which are liquid at room temperature, of aluminum compounds of the general formula V as claimed in claim 4, which comprises heating aluminum alcoholates of the general formula VI
Al (O-R⁶)₃ (VI),
in which R⁶ is an alkyl group having 2 to 10 carbon atoms,
with at least 3 mol of an alkyl acetoacetate of an alcohol different from R⁶OH per mole of aluminum alcoholate, without removing the alcohol liberated in the reaction during the heating from the reaction mixture.

6. A process for preparing the mixtures, which are liquid at room temperature, of aluminum compounds of the general formula V as claimed in claim 4, which comprises heating aluminum alcoholates of the general formula VI
Al (O-R⁶)₃ (VI),
in which R⁶ is an alkyl group having 2 to 10 carbon atoms, with at least 3 mol of a mixture of at least 2 different alkyl acetoacetates.

7. A continuous process for the preparation of the mixtures, which are liquid at room temperature, of aluminum compounds of the general formula V, as claimed in claim 4, which comprises reacting in each case one mol of an aluminum alcoholate of the general formula VI
Al (O-R⁶)₃ (VI),
in which R⁶ is an alkyl group having 2 to 10 carbon atoms, and 3 to 10 mol of an alkyl acetoacetate of the general formula III
CH₃-CO-CH₂-COOR² (III),
in which R² is alkyl having 1 to 5 carbon atoms,
where R² and R⁶ are different, or else with at least 3 mol of a mixture of 2 or 3 different alkyl acetoacetates of the general formula III in pure form or dissolved in a suitable inert solvent, continuously
at temperatures from 100 to 250°C,
under a pressure of from 1 to 100 bar, and
with residence times of from 5 to 120 minutes,
it being necessary to choose the pressure and temperature so that no gas phase can form in the reaction vessel.

8. A process as claimed in claim 7, wherein the aluminum alcoholate of the general formula VI is mixed with the alkyl acetoaceate or the alkyl acetoacetates of the general formula III continuously under elevated pressure and at elevated temperature in a mixer and then reacted in a reactor with tubular characteristics.

9. A process as claimed in any of claims 1 to 3, wherein R³ is the group -CH₃, -C₂H₅ or -CH(CH₃)-C₂H₅, R⁴ is -CH(CH₃)₂, -CH(CH₃)-C₂H₅ or -CH-(CH₃)C₃H₇, R⁵ is CH₃, -C₂H₅, CH(CH₃)₂, CH(CH₃)-C₂H₅ or -CH-(CH₃)C₃H₇.

10. A process as claimed in any of claims 5 to 7, wherein R⁶ is -CH₂-CH(CH₃)₂, -CH(CH₃)-C₂H₅ or -CH(CH₃)-C₃H₇.

11. A process as claimed in claim 7, wherein R² is -CH₃, -C₂H₅, -CH(CH₃)-C₂H₅ or -CH₂-CH(CH₃)₂.

## Revendications

1. Procédé pour la préparation de cétones gamma, delta-insaturées répondant à la formule générale I 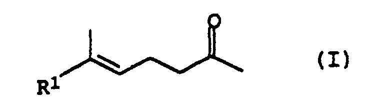 par réaction d'un alcool allylique de formule générale II 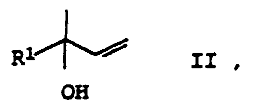 dans laquelle R¹ représente H ou un radical hydrocarboné ramifié, saturé ou insaturé, en C1-C20, avec le dicétène ou un acétylacétate d'alkyle de formule générale III 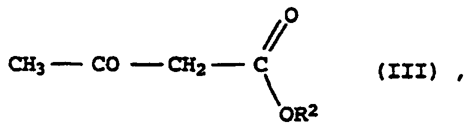 dans laquelle R² représente un groupe alkyle en C1-C5, dans une réaction de Carroll éventuellement modifiée, en présence d'un catalyseur à base d'aluminium, **caractérisé par le fait que** l'on utilise en tant que catalyseur à base d'aluminium un dérivé de l'aluminium de formule générale V ou un mélange de dérivés de l'aluminium de formule générale V 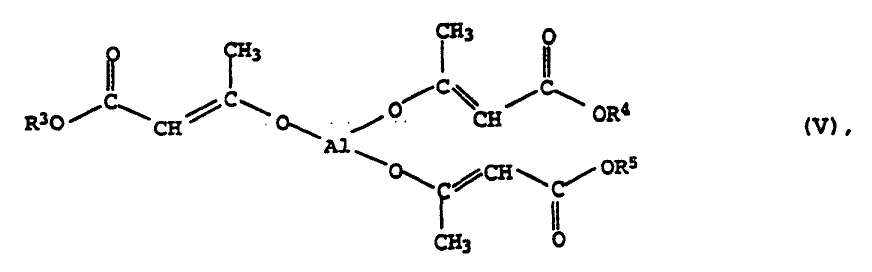 dans laquelle
R³ représente un groupe alkyle en C1-C5,
R⁴ représente un groupe alkyle en C3-C 10 et
R⁵ représente un groupe alkyle en C1-C10,
les groupes R³, R⁴ et R⁵ ne devant pas être identiques.

2. Procédé selon la revendication 1, **caractérisé par le fait que** l'on utilise en tant que catalyseur à base d'aluminium un mélange de dérivés de l'aluminium de formule générale V 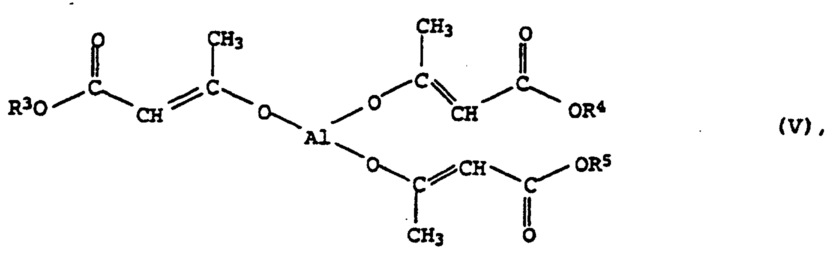 dans laquelle 3
R représente un groupe alkyle en C1-C5,
R⁴ représente un groupe alkyle en C3-C10 et
R⁵ représente un groupe alkyle en C1-C10,
les symboles R³, R⁴ et R⁵ ne pouvant avoir, tous trois, une signification identique.

3. Procédé selon la revendication 1, **caractérisé par le fait que** l'on fait réagir avec l'acétylacétate d'alkyle de formule III un alcool allylique de formule générale II qui consiste en le 2-méthyl-3-butèn-2-ol, le linalol, le 6,7-dihydro-linalol, le nérolidol, le 10,11-dihydro-nérolidol ou le géranylinalol.

4. Mélanges de dérivés de l'aluminium liquides à température ambiante, répondant à la formule générale V 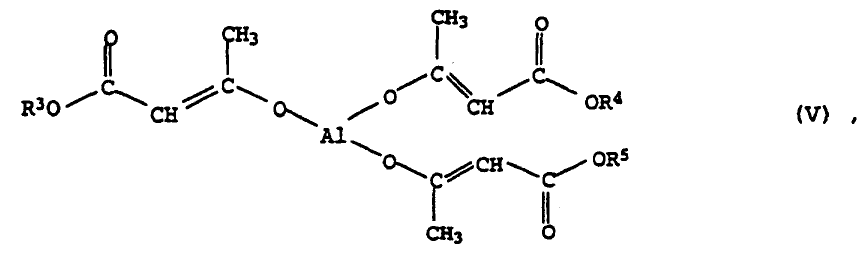 dans laquelle
R³ représente un groupe alkyle en C1-C5,
R⁴ représente un groupe alkyle en C3-C10 et
R⁵ représente un groupe alkyle en C1-C10,
les symboles R³, R⁴ et R⁵ ne pouvant avoir tous trois, la même signification.

5. Procédé pour la préparation de mélanges liquides à température ambiante de dérivés de l'aluminium de formule générale V selon la revendication 4, **caractérisé par le fait que** l'on chauffe des alcoolates d'aluminium de formule générale VI
Al(O-R⁶)₃ (VI)
dans laquelle R⁶ représente un groupe alkyle en C2-C10, avec au moins 3 mol d'un acétylacétate d'alkyle d'un alcool autre que R⁶0H par mol de l'alcoolate d'aluminium sans éliminer du mélange de réaction, en cours de réaction, l'alcool libéré dans la réaction.

6. Procédé pour la préparation de mélanges liquides à température ambiante de dérivés de l'aluminium de formule générale V selon la revendication 4, **caractérisé par le fait que** l'on chauffe des alcoolates d'aluminium de formule générale VI
Al(O-R⁶)₃ (VI)
dans laquelle R⁶ représente un groupe alkyle en C2-C10, avec au moins 3 mol d'un mélange d'au moins deux acétylacétates d'alkyle différents.

7. Procédé continu pour la préparation des mélange liquides à température ambiante de dérivés de l'aluminium de formule générale V selon la revendication 4, **caractérisé par le fait que** l'on fait agir à des températures de 100 à 250° C, sous une pression de 1 à 100 bar et dans des durées de contact de 5 à120 minutes, 1 mol d'un alcoolate d'aluminium de formule générale VI
Al(O-R⁶)₃ (VI)
dans laquelle R⁶ représente un groupe alkyle en C2-C10 et 3 à 10 mol d'un acétylacétate d'alkyle de formule générale II
CH₃-CO-CH₂-COOR² (III)
dans laquelle R² représente un groupe alkyle en C1-C5,
R² et R⁶ ayant des significations différentes, ou bien avec au moins 3 mol d'un mélange de deux ou trois acétylacétates d'alkyle différents de formule générale III, à l'état pur ou à l'état de solutions dans un solvant approprié, en réglant la pression et la température en sorte qu'il ne puisse pas se former de phase gazeuse dans le récipient de réaction.

8. Procédé selon la revendication 7, **caractérisé par le fait que** l'on envoie ensemble, en continu, sous haute pression et à haute température, l'alcoolate d'aluminium de formule générale VI avec l'acétylacétate d'alkyle ou les acétylacétates d'alkyle de formule générale III dans un mélangeur, puis on les fait réagir dans un appareil à caractéristique tubulaire .

9. Procédé selon l'une des revendications 1 à 3, **caractérisé par le fait que** R³ représente -CH₃, -C₂H₅ ou CH(CH₃)-C₂H₅, R⁴ représente -CH(CH₃)₂, -CH(CH₃)-C₂H₅ ou -CH-(CH ₃) C₃H₇, et R⁵ représente CH₃, -C₂H₅, CH(CH₃)₂, CH(CH₃)-C₂H₅ ou -CH-(CH₃)C₃H₇.

10. Procédé selon l'une des revendications 5 à 7, **caractérisé par le fait que** R⁶ représente -CH₂-CH(CH₃)₂. -CH(CH₃)-C₂H₅ ou -CH(CH₃)C₃H₇.

11. Procédé selon revendication 7, **caractérisé en ce que** R² représente -CH₃, -C₂H₅, -CH(CH₃)-C₂H₅ ou -CH₂-CH(CH₃)₂.
